(19)

Europäisches
Patentamt
European
Patent Office
Office européen
des brevets

(11) **EP 2 476 423 A1**

(12) **EUROPEAN PATENT APPLICATION**
published in accordance with Art. 153(4) EPC

(43) Date of publication:
    **18.07.2012 Bulletin 2012/29**

(21) Application number: **10815256.2**

(22) Date of filing: **20.08.2010**

(51) Int Cl.:
    **A61K 33/08** (2006.01)      **A61K 9/20** (2006.01)
    **A61K 47/12** (2006.01)      **A61K 47/32** (2006.01)
    **A61K 47/38** (2006.01)      **A61P 1/04** (2006.01)
    **A61P 1/10** (2006.01)

(86) International application number:
    **PCT/JP2010/064066**

(87) International publication number:
    **WO 2011/030659 (17.03.2011 Gazette 2011/11)**

(84) Designated Contracting States:
    **AL AT BE BG CH CY CZ DE DK EE ES FI FR GB
    GR HR HU IE IS IT LI LT LU LV MC MK MT NL NO
    PL PT RO SE SI SK SM TR**

(30) Priority: **08.09.2009 JP 2009206720**

(71) Applicant: **KYOWA CHEMICAL INDUSTRY CO.,
    LTD.
    Kagawa-ken 761-0113 (JP)**

(72) Inventors:
    • **KITAJIMA, Hideaki
      Kita-gun
      Kagawa 761-0705 (JP)**
    • **HORIE, Shiro
      Kita-gun
      Kagawa 761-0705 (JP)**

    • **KUBO, Takaaki
      Kita-gun
      Kagawa 761-0705 (JP)**
    • **KAWANABE, Naruhito
      Kita-gun
      Kagawa 761-0705 (JP)**
    • **ANABUKI, Tomotaka
      Kita-gun
      Kagawa 761-0705 (JP)**

(74) Representative: **Raynor, Stuart Andrew et al
    J A Kemp
    14 South Square
    Gray's Inn
    London WC1R 5JJ (GB)**

(54) **ANTACID AND LAXATIVE TABLET**

(57)    A tablet which can sustain excellent disintegration properties (may be referred to as "quick disintegration properties" hereinafter), as a tableted product, for a long time and has excellent shape-retention stability by the improvement of the strength of the tablet.

The antacid and laxative tablet comprises magnesium oxide particles as the main component, wherein (a) the average secondary particle diameter measured by a laser diffraction scattering method of the magnesium oxide particles is 0.5 to 10 $\mu$m, (b) the content of the magnesium oxide particles is 85 to 96 wt%, (c) the content of crystalline cellulose as a binder is 2 to 8 wt%, (d) the content of croscarmellose sodium as a disintegrating agent-I is 0.8 to 2.5 wt%, (e) the content of insoluble polyvinyl pyrrolidone as a disintegrating agent-II is 1 to 3.5 wt%, and (f) the content of a lubricant is 0.5 to 2 wt%.

Fig. 1

EP 2 476 423 A1

**Description**

Technical Field:

The present invention relates to an antacid and laxative tablet comprising magnesium oxide particles as the main component. More specifically, it relates to an antacid and laxative tablet which has a high content of magnesium oxide particles of not less than 85 wt%, a short disintegration time of 10 seconds or less and sustains its short disintegration time characteristic for a long time. The present invention also relates to an antacid and laxative tablet which has excellent shape-retention stability with extremely low susceptibility to wear and chipping though it has a high content of magnesium oxide particles. The present invention further relates to a process of producing an antacid and laxative tablet comprising magnesium oxide particles as the main component and having the above-described characteristic properties.

Background Art:

Heretofore, a tablet comprising magnesium oxide particles as the main component has been known as an antacid or laxative tablet, and the amount thereof is growing. This tablet comprising magnesium oxide particles is produced by mixing additives such as a binder and a disintegrating agent with magnesium oxide particles and tableting the resulting mixture. The Applicant of the present invention previously proposed a tablet which has a short disintegration time of 10 seconds or less and accordingly is easily taken when it is administered and has a high content of magnesium oxide particles (refer to Patent Document 1).

The tablet of this Patent Document 1 contains 88 to 97 wt% of magnesium oxide particles having a specific average secondary particle diameter, 1 to 10 wt% of crystalline cellulose or starch as a binder and 1 to 3. 5 wt% of croscarmellose sodium or carboxy starch sodium as a disintegrating agent. Since this tablet has composition that hard magnesium oxide particles are easily tableted and has a disintegration time of 10 seconds or less, it is easily taken.

Prior Art Documents:

Patent Documents:

Patent Document 1: JP-A 2003-146889

Outline of the Invention:

Problems that the Invention is to Solve:

The tablet of the above Patent Document 1 is an excellent magnesium oxide-containing tablet which is easily tableted and easily taken. However, it was found that when this tablet is kept in a dry state, its excellent disintegration properties are sustained for a relatively long time whereas when it is kept at normal temperature in a wet condition, its disintegration time is extended. Especially when the tablet is kept under the conditions of an acceleration test (40°C, RH of 75 %) which are required for tablets (bare tablets), it was found that the short disintegration time characteristic of the tablet is impaired in 4 to 6 days. Although the reason for this is unknown, it is assumed that some of magnesium oxide particle crystals on the surface of the tablet absorb moisture under a high humidity condition and change into magnesium hydroxide having poor solubility and that the disintegration agent component is thickened by the absorption of moisture.

Although the above tablet has an advantage that it is easily tableted and molded, the cracking, wear and chipping of the obtained tablet occur in no small measure and the improvement of these is desired.

Then, the inventors of the present invention conducted studies with a view to providing a tablet which can sustain excellent disintegration properties (may be referred to as "quick disintegration properties" hereinafter) as a tableted product for a long time and has excellent shape-retention stability by the improvement of the strength of the tablet.

As a result, they found that when specific two different compounds are used as disintegrating agents in a specific ratio and in combination with a specific binder, the obtained tablet sustains its quick disintegration properties for a long time even when it is kept under an ordinary wet condition. The inventors of the present invention assume that the reason for this is probably that the two different disintegrating agents and the binder interact with each other to prevent some of the magnesium oxide particle crystals from changing into magnesium hydroxide and the disintegrating agents from becoming thickened.

According to studies conducted by the inventors of the present invention, it was found that a tablet which rarely experiences a tableting trouble and has excellent shape-retention stability with low susceptibility to wear and chipping is obtained when the tablet has a specific shape. It is considered that this shape-retention stability effect is due to not only the specific shape but also a combination of the two different disintegrating agents and the binder.

Means for Solving the Problems:

According to the present invention, there is provided an antacid and laxative tablet comprising magnesium oxide particles as the main component, wherein (a) the average secondary particle diameter measured by a laser diffraction scattering method of the magnesium oxide particles is 0.5 to 10 μm, (b) the content of the magnesium oxide particles is 85 to 96 wt%, (c) the content of crystalline cellulose as a binder is 2 to 8 wt%, (d) the content of croscarmellose sodium as a disintegrating agent-I is 0.8 to 2.5 wt%, (e) the content of insoluble polyvinyl pyrrolidone as a disintegrating agent-II is 1 to 3.5 wt%, and (f) the content of a lubricant is 0.5 to 2 wt%.

Preferably, the antacid and laxative tablet of the present has the following features.

(1) The magnesium oxide particles have an average secondary particle diameter measured by the laser diffraction scattering method of 1 to 7 μm.
(2) The content of the magnesium oxide particles is 88 to 92 wt%.
(3) The content of crystalline cellulose as the binder is 4 to 6 wt%.
(4) The content of croscarmellose sodium as the disintegrating agent-I is 1.2 to 1.8 wt%.
(5) The content of insoluble polyvinyl pyrrolidone as the disintegrating agent-II is 1.5 to 2.8 wt%.
(6) The lubricant is calcium stearate.
(7) The antacid and laxative tablet has a domed shape on each of the upper and lower horizontal surfaces of a cylindrical shape, the domed shape on each of the upper and lower horizontal surfaces satisfying the following requirements (a), (b) and (c) in the cross-section shape including the center line of the cylindrical shape:

(a) the corner angle should be 25 to 45°,
(b) the corner horizontal length should be 0.30 to 1.0 mm, and
(c) the cup depth should be 0.6 to 1.2 mm.

(8) The corner angle (a) in the above cross-section shape is 28 to 40°.
(9) The corner horizontal length (b) in the above cross-section shape is 0.35 to 0.85 mm.
(10) The cup depth (c) in the above cross-section shape is 0.65 to 1.1 mm.
(11) The length (diameter) of the horizontal surface in the above cross-section shape is 7 to 11 mm.

According to the present invention, there is provided a process of producing an antacid and laxative tablet comprising magnesium oxide particles as the main component, comprising the steps of:

(1) mixing together magnesium oxide particles having an average secondary particle diameter measured by a laser diffraction scattering method of 0. 5 to 10 μm, crystalline cellulose as a binder, croscarmellose sodium as a disintegrating agent-I and a lubricant, and dry granulating the resulting mixture to produce magnesium oxide granules;
(2) blending crystalline cellulose as a binder, insoluble polyvinyl pyrrolidone as a disintegrating agent-II and a lubricant with the magnesium oxide granules; and
(3) tableting the obtained granules.

The above magnesium oxide granules have an average particle diameter of 250 to 550 μm.

Effects of the Invention:

According to the present invention, there is provided an antacid and laxative tablet which has a high content of magnesium oxide particles, disintegrates in the oral cavity in a short time when it is taken, sustains its quick disintegration properties for a long time and therefore is easily taken. In addition, the tablet of the present invention is easily tableted, and the obtained tablet has excellent shape-retention stability with extremely low susceptibility to wear and chipping. According to the present invention, there is also provided a process of producing the above tablet.

Brief Description of the Drawings:

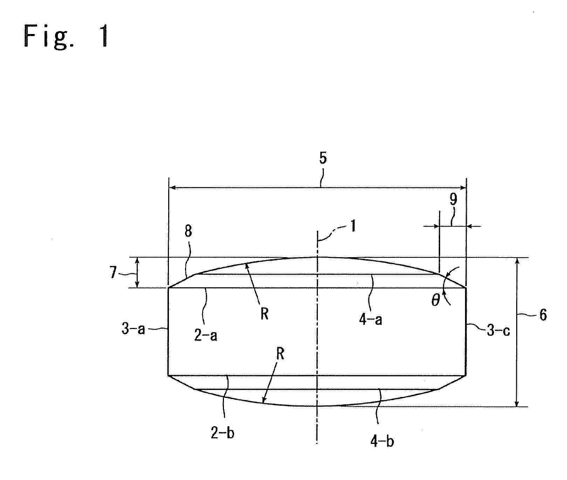

Fig. 1 shows the tablet of the present invention having a preferred shape which has a domed shape on each of the upper and lower horizontal surfaces of a cylindrical shape. This figure shows the vertical-section structure of the tablet along the center line.

Mode for Carrying Out the Invention:

The antacid and laxative tablet of the present invention and a production process thereof will be described in more detail hereinunder.

The magnesium oxide particles used in the present invention have an average secondary particle diameter measured by a laser diffraction scattering method of 0.5 to 10 μm, preferably 1 to 7 μm. A tablet having a high content of magnesium oxide particles of 85 to 96 wt%, preferably 88 to 92 wt% is obtained by using magnesium oxide particles having this particle diameter and a combination of the above specific binder and the above specific disintegrating agents.

Although the magnesium oxide particles to be tableted may be powdery or granular, the particles are preferably granular because a tablet which has an excellent effect of preventing the abrasion of a tableting machine, more excellent shape-retention stability and a high content of magnesium oxide particles can be obtained.

The magnesium oxide particles are generally obtained by baking magnesium hydroxide particles. The magnesium oxide particles obtained by baking magnesium hydroxide having an average secondary particle diameter measured by a laser diffraction scattering method of 1 to 10 μm at 700 to 1,000°C have the above average secondary particle diameter and provide a tablet having excellent disintegration properties when they are tableted.

In the tablet of the present invention, crystalline cellulose is used as a binder. The crystalline cellulose is used in the tablet in an amount of 2 to 8 wt%, preferably 4 to 6 wt%.

In the tablet of the present invention, two different types of disintegrating agents are used. That is, croscarmellose sodium is used as a disintegrating agent-I and insoluble polyvinyl pyrrolidone is used as a disintegrating agent-II. The croscarmellose sodium as the disintegrating agent-I is used in the tablet in an amount of 0.8 to 2.5 wt%, preferably 1.2 to 1.8 wt%. The insoluble polyvinyl pyrrolidone as the disintegrating agent-II is used in the tablet in an amount of 1 to 3.5 wt%, preferably 1.5 to 2.8 wt%. The insoluble polyvinyl pyrrolidone as the disintegrating agent-II is obtained by crosslinking soluble polyvinyl pyrrolidone and insolubilizing it. The term "insoluble" means that vinyl pyrrolidone is hardly soluble or insoluble in water, methanol, ethanol or diethyl ether. When the soluble polyvinyl pyrrolidone is used as a disintegrating agent in the tablet of the present invention, it has no effect of sustaining quick disintegration properties. The insoluble polyvinyl pyrrolidone as the disintegrating agent -II is marketed under the generic name of crospovidone, specifically, the trade name of Kollidone CL from BASF Japan AG.

A lubricant is used in the tablet of the present invention.
Examples of the lubricant include stearic acid and salts (Na, Mg and Ca salts) thereof. Stearic acid salts are preferred, and calcium stearate and magnesium stearate are particularly preferred. Out of these, calcium stearate is the most effective. When the amount of the lubricant is too large, disintegration is delayed and when the amount of the lubricant is too small, the lubricant adheres to a pestle or a mortar. Therefore, the amount of the lubricant is preferably 0.5 to 2 wt%, more preferably 0.8 to 1.5 wt%.

To produce the tablet of the present invention, the above-described magnesium oxide particles, binder, disintegrating agent-I, disintegrating agent-II and lubricant are mixed together in a predetermined ratio, and the resulting mixture is tableted. It is preferred that granules should be produced and tableted as will be described hereinafter.

The tableting pressure is preferably 5 to 12 kN, more preferably 6 to 10 kN as a punch pressure per tablet. The shape of the pestle may have a corner angle R, corner angle-flat plane or round corner-flat plane besides the R-surface.

The tablet of the present invention can contain magnesium oxide particles in a high ratio and is easily taken because it disintegrates quickly in the mouth when it is taken with water.

The tablet of the present invention has excellent shape-retention stability, and its quick disintegration properties are maintained for a long time as a result of an acceleration test which will be described hereinafter.

The tablet of the present invention does not differ from ordinary tablets for oral use in size and shape. The tablet of the present invention has a diameter of 5 to 12 mm, preferably 6 to 10 mm, particularly preferably 6 to 9 mm. The tablet has a thickness of 2 to 6 mm, preferably 2 to 5 mm, particularly preferably 2.5 to 4.5 mm. Further, the weight of each tablet is 100 to 1,000 mg, preferably 150 to 800 mg, most preferably 200 to 600 mg.

According to studies conducted by the inventors of the present invention, it was found that a tablet which is particularly excellent in shape-retention stability and quick disintegration properties is obtained by mixing together the above components by the method which will be described hereinafter to obtain granules and tableting the obtained granules.

That is, according to the present invention, there is provided the following process of producing the tablet. The process of producing an antacid and laxative tablet comprising magnesium oxide particles as the main component, comprising the steps of:

(1) mixing together magnesium oxide particles having an average secondary particle diameter measured by a laser diffraction scattering method of 0.5 to 10 μm, crystalline cellulose as a binder, croscarmellose sodium as a disintegrating agent-I and a lubricant, and dry granulating the resulting mixture to produce magnesium oxide granules;
(2) blending crystalline cellulose as a binder, insoluble polyvinyl pyrrolidone as a disintegrating agent-II and a lubricant with the magnesium oxide granules; and
(3) tableting the obtained granules.

The above production process is characterized in that the granules are produced and then tableted, the disintegrating agent-I is blended at the time of producing the granules, and the disintegrating agent-II is blended after the granules are obtained. The crystalline cellulose is blended twice at the time of producing the granules and after the granules are produced. These crystalline cellulose's may be the same or different. The average particle diameter of the granules is 250 to 550 μm, preferably 300 to 500 μm.

Further, according to studies conducted by the inventors of the present invention, it was found that when the tablet has the following shape, it is easily tableted, rarely chipped and extremely excellent in shape-retention stability. The obtained tablet has low susceptibility to wear and chipping.

That is, according to the present invention, there is provided an antacid and laxative tablet (may be referred to as "tablet A" hereinafter) which has a domed shape on each of the upper and lower horizontal surfaces of a cylindrical shape as a preferred shape of the tablet, the domed shape on each of the upper and lower horizontal surfaces satisfying the following requirements (a), (b) and (c) in the cross-section shape including the center line of the cylindrical shape:

(a) the corner angle should be 25 to 45°, preferably 28 to 40°,
(b) the corner horizontal length should be 0.30 to 1.0 mm, preferably 0.35 to 0.85 mm, and
(c) the cup depth should be 0.6 to 1.2 mm, preferably 0.65 to 1.1 mm.

The preferred shape of the tablet A of the present invention will be described with reference to the accompanying drawing.
Fig. 1 shows the cross-section structure in the vertical direction along the center line 1 of the cylindrical shape of the tablet A. As shown in Fig. 1, the cross-section structure in the vertical direction along the center line of the tablet A (may be simply referred to as "cross-section shape" hereinafter) is bilaterally symmetric about the center line as the central axis, and the cylindrical shape has a basic skeleton composed of four sides which are an upper horizontal surface 2-a, a lower horizontal surface 2-b, a surrounding side surface 3-a and a surrounding side surface 3-b and also a domed shape on the upper horizontal surface 2-a of this basic skeleton and a domed shape on the lower horizontal surface 2-b. The domed shape on the upper horizontal surface and the domed shape on the lower horizontal surface are symmetric in the vertical direction and identical.

The tablet A of the present invention has the following features (a) to (e) in the cross-section shape:

(a) the tablet has a quadrilateral basic skeleton based on a cylindrical shape (the basic skeleton portion has a round disk-like shape),
(b) the angle θ between each of the linear sides 8 of the domed shape from the end of the upper horizontal surface 2-a and the horizontal surface (to be referred to as "corner angle") is 25 to 45°,
(c) the distance 9 projected on the upper horizontal surface of the length of each of the linear sides 8 having the above corner angle (to be referred to as "corner horizontal length") is 0.35 to 0.85 mm,
(d) the length 7 of the center line from the upper horizontal surface to the apex of the domed shape (to be referred to as "cup depth") is 0.65 to 1.1 mm, and
(e) the tablet is bilaterally symmetric about the center line 1 as the central axis, and the domed shapes on the upper horizontal surface and on the lower horizontal surface are symmetric in the vertical direction.

Further, in the cross-section shape of the tablet of the present invention, the domed shape formed on an upper dome horizontal surface 4-a is desirably part of a circle having a single curvature R (to be referred to as "curvature R"), and the radius of the curvature R is 7 to 25 mm, preferably 8 to 22 mm.

The tablet A of the present invention is characterized in that it has a cross-section shape and lengths shown in (a) to (e).
Out of these, the corner angle θ is 25 to 45°, preferably 28 to 40°, particularly preferably 29 to 33°. It is important that the sides 8 from both ends of the horizontal surfaces forming this corner angle should be linear and should have a fixed length.

That is, the cross-section shape of the tablet A of the present invention is characterized in that each of the domed shapes formed on the upper and lower horizontal surfaces has a two-stage structure consisting of linear portions (linear sides 8) from both ends of the horizontal surface and part of a circle having a curvature R.

The tablet which has a strong shape as air is smoothly removed therefrom by the compression of the solid particles at the time of tableting due to this two-stage structure has extremely low susceptibility to wear and chipping caused by an impact and contact between tablets. When a tablet having domed shapes which have a curve directly from both ends of the horizontal surfaces is made, even if the angle of each of the domed shapes is about 30° , the removal of air does not occur fully and a tablet which is susceptible to wear and chipping may be obtained.

The tablet A of the present invention has a corner horizontal length 9 of 0.3 to 1.0 mm, preferably 0.35 to 0.85 mm in the cross-section shape. The cup depth 7 is 0.6 to 1.2 mm, preferably 0.65 to 1.1 mm.

The tablet A of the present invention comprises magnesium oxide particles as the main component and has a weight of about 200 to about 600 mg each. As for the size of the tablet of the present invention, the weight of the tablet can be changed by varying its thickness and diameter. The diameter 5 (the length of each of the upper and lower horizontal surfaces) of the tablet is 6 to 14 mm, preferably 7 to 11 mm, and the thickness 6 of the tablet is 4 to 6 mm, preferably 4.2 to 5.6 mm. The diameter 5 and the thickness 6 may be suitably determined from the above ranges based on the desired weight of each tablet.

According to the present invention, there can be provided a tablet which has a high content of magnesium oxide particles, is compression tableted and has excellent shape-retention stability with extremely low susceptibility to wear and chipping in compression tableting, transport, storage, packaging and sales processes by specifying its shape. The magnesium oxide particle-containing tablet of the present invention can have a high content of magnesium oxide particles, is extremely excellent in shape stability, is easily taken as its disintegration time is very short when it is orally administered and sustains its disintegration properties.

The tablet of the present invention is orally administered for antacid or laxative purpose. The dose of the tablet differs according to purpose and disease conditions. Normally, 2 g is administered to each adult each day. This 2 g is equivalent to 6 to 8 tablets on average and divided into 1 to 3 parts to be administered each day.

EXAMPLES

The following examples are provided to further illustrate the tablet of the present invention. In Examples and Comparative Example, (a) hardness, (b) disintegrating time, (c) degree of friability and (d) thickness of the tablet, and (e) particle size distribution of granules, (f) average particle diameter of granules and (g) bulk density of granules are values measured by the following methods.

(a) Hardness of tablet

The hardness of the tablet was measured by using the DC-50 tablet hardness meter (of Okada Seiko Co., Ltd.).

(b) Disintegration time

This was measured by using water as a test liquid in accordance with the "general test method/disintegration test method of the 15th Revised Japanese Pharmacopoeia test methods".

(c) Degree of friability

The degree of friability (percentage of a mass loss to an initial mass) was calculated after 100 revolutions (24 to 26 rpm) by using the number of tablets equivalent to an amount as close to a total mass of 6.5 g as possible as test samples.
This was measured in accordance with the "reference information/tablet friability degree test method of the 15th Revised Japanese Pharmacopoeia test methods".

$$\text{Degree of friability (\%)} = \frac{《 <i> - <ii> 》 \times 100}{<iii>}$$

<i>: initial mass (g) of tablet before friability test
<ii>: mass (g) of tablet after friability test
<iii>: 100/initial mass (g) of tablet before friability test

(d) Thickness of tablet

The thickness of the tablet was measured by using a thickness gauge (of Niigata Seiki Co., Ltd.).

(e) Particle size distribution of granules

Electromagnetic vibration sieve machine: Octagon of Endecots Co., Ltd.
Used sieves: 850, 710, 500, 355, 180, 150, 106 $\mu$m
Test conditions: vibration strength of 5, sieving time of 5 minutes

About 100 g of the sample was put on the top sieve of a container containing a receptor and sieves placed on the receptor, a top cover was put on the container, and the container was set in the machine. After it was tested under the above conditions, the weight of the residue remaining on each of the sieves and the receptor was measured (down to 0.01 g).

(f) Average particle diameter D50 of granules

This is a particle diameter which is a 50 wt% integrated value obtained by integrating from the particle diameters of large particles in the above particle size distribution.

(g) Bulk density of granules

The sample was put through a standard sieve of 16 mesh (opening of 1.0 mm) into a container having a volume of 100 ml used in the apparent specific volume standing method in accordance with JIS K5101 until it was heaped up. This heap was chipped off linearly with a spatula.
The mass of the content of the receptor was weighed down to 0.01 g.

$$\text{Bulk density (g/ml)} = F/100$$

(F: mass of sample in receptor, 100: volume of receptor (ml))

Examples of pharmaceutical formulation

The following production process was carried out in accordance with formulation examples shown in Tables 1, 2 and 3.
Basically, 330 mg preparations and 250 mg preparations are based on almost the same proportional formulation as 500 mg preparations.
The product names (or trade names) of reagents used in Examples and Comparative Examples and their manufacturers are as follows.

(a) Magnesium oxide particles: "Magnesium Oxide T" (of Kyowa Chemical Industry, Co., Ltd.)
(b) Crystalline cellulose-I: "VIVAPUR 101 Premium" (of JRS Pharma Gmbh & C0., Kg.)
(c) Crystalline cellulose-II: "Theorus KG-1000" (of Asahi Kasei Chemicals Co., Ltd.)
(d) Corn starch: "purified dry sterilized corn starch" (of Matsuya Kagaku Kogyo Co., Ltd.)
(e) Croscarmellose sodium: "KIkkorate ND-2HS" (of Nichirin Kagaku Kogyo Co., Ltd.)
(f) Calcium stearate: "plant-based calcium stearate" (of Taihei Kagaku Sangyo Co., Ltd.)
(g) Insoluble polyvinyl pyrrolidone (crospovidone): "Kollidon CL" (of BASF Japan AG)

In Tables 1 to 3, "internal additives" means reagents which were used when granules were prepared, and "external additives" means reagents which were blended with the obtained granules (shaped granules).

Table 1

500 mg pharmaceutical formulation of magnesium oxide tablet

| Reagents | volume of prescription (mg) | | |
|---|---|---|---|
| | Ex. 1 | Ex. 2 | Comp. Ex. 1 |
| Internal additives | | | |

(continued)

500 mg pharmaceutical formulation of magnesium oxide tablet

| Reagents | | volume of prescription (mg) | | |
|---|---|---|---|---|
| | | Ex. 1 | Ex. 2 | Comp. Ex. 1 |
| | Magnesium oxide | 515.00 | 515.00 | 520.00 |
| | Crystalline cellulose-I | 10.00 | 5.00 | 36.00 |
| | Crystalline cellulose-II | 10.00 | 15.00 | - |
| | Croscarmellose sodium | 8.00 | 8.00 | 18.00 |
| | Calcium stearate | 2.00 | 2.00 | - |
| External additives | | | | |
| | Calcium stearate | 5.00 | 5.00 | 6.00 |
| | Crospovidone | 10.00 | 15.00 | - |
| | Crystalline cellulose-II | 5.00 | 5.00 | - |
| Total | | 570.00 | 570.00 | 580.00 |

Table 2

330 mg pharmaceutical formulation of magnesium oxide tablet

| Reagents | | Volume of prescription (mg) | | |
|---|---|---|---|---|
| | | Ex. 3 | Ex. 4 | Comp. Ex. 2 |
| Internal additives | | | | |
| | Magnesium oxide | 339.90 | 339.90 | 330.00 |
| | Crystalline cellulose-I | 6.60 | 3.30 | 23.00 |
| | Crystalline cellulose-II | 6.60 | 9.90 | - |
| | Corn starch | - | - | 7.00 |
| | Croscarmellose sodium | 5.28 | 5.28 | 11.00 |
| | Calcium stearate | 1.32 | 1.32 | - |
| External additives | | | | |
| | Calcium stearate | 3.30 | 3.30 | 4.00 |
| | Crospovidone | 9.90 | 9.90 | - |
| | Crystalline cellulose-II | 3.10 | 3.10 | - |
| Total | | 376.00 | 376.00 | 375.00 |

Table 3

250 g pharmaceutical formulation of magnesium oxide tablet

| Reagents | | Volume of prescription (mg) | | |
|---|---|---|---|---|
| | | Ex. 5 | Ex. 6 | Comp. Ex. 3 |
| Internal additives | | | | |
| | Magnesium oxide | 257.50 | 257.50 | 250.00 |

(continued)

250 g pharmaceutical formulation of magnesium oxide tablet

| | | Volume of prescription (mg) | | |
|---|---|---|---|---|
| Reagents | | Ex. 5 | Ex. 6 | Comp. Ex. 3 |
| | Crystalline cellulose-I | 5.00 | 2.50 | 18.00 |
| | Crystalline cellulose-II | 5.00 | 7.50 | - |
| | Corn starch | - | - | 5.00 |
| | Croscarmellose sodium | 4.00 | 4.00 | 9.00 |
| | Calcium stearate | 1.00 | 1.00 | - |
| External additives | | | | |
| | Calcium stearate | 2.50 | 2.50 | 3.00 |
| | Crospovidone | 7.50 | 7.50 | - |
| | Crystalline cellulose-II | 2.50 | 2.50 | - |
| Total | | 285.00 | 285.00 | 285.00 |

Examples 1, 3 and 5: (500 mg, 300 mg and 250 mg preparations)

17.17 kg of magnesium oxide particles having an average secondary particle diameter of 6.50 $\mu$m, 0.33 kg of crystalline cellulose-I, 0.33 kg of crystalline cellulose-II having high moldability, 0.27 g of croscarmellose sodium and 0.07 kg of calcium stearate were mixed together by means of a container type mixer, the resulting mixture was granulated at a roll pressure of 5 MPa by means of a roll molding type dry granulating machine to obtain a molded product, and granules were produced from the molded product by means of an oscillator type grinder. 18.17 kg of the shaped granules and 0.17 kg of calcium stearate were mixed together by means of a container type mixer, and the resulting mixture was further mixed with 0.50 g of crospovidone and 0.17 kg of crystalline cellulose-II as external additives by means of a container type mixer to obtain granules to be tableted (see Table 4). A 330 mg preparation contained 0.16 kg of crystalline cellulose-II as an external additive. The physical properties of the granules to be tableted are shown in Tables 5, 6 and 7.

Examples 2, 4 and 6: (500 mg, 330 mg and 250 mg preparations)

17.17 kg of magnesium oxide particles having an average secondary particle diameter of 6.50 $\mu$m, 0.17 kg of crystalline cellulose-I, 0.50 kg of crystalline cellulose-II, 0.27 g of croscarmellose sodium and 0.07 kg of calcium stearate were mixed together by means of a container type mixer, the resulting mixture was granulated at a roll pressure of 5 MPa by means of a roll molding type dry granulating machine to obtain a molded product, and granules were produced from the molded product by means of an oscillator type grinder. 18.17 kg of the shaped granules and 0.17 kg of calcium stearate were mixed together by means of a container type mixer, and the resulting mixture was further mixed with 0.50 kg of crospovidone and 0.17 kg of crystalline cellulose-II as external additives by means of a container type mixer to obtain granules to be tableted (see Table 4). A 330 mg preparation contained 0.16 kg of crystalline cellulose-II as an external additive. The physical properties of the granules to be tableted are shown in Tables 5, 6 and 7.

Comparative Example 1: (500 mg preparation)

17.17 kg of magnesium oxide particles having an average secondary particle diameter of 6.50 $\mu$m, 1.19 kg of crystalline cellulose-I and 0.59 g of croscarmellose sodium were mixed together by means of a container type mixer, the resulting mixture was granulated at a roll pressure of 5 MPa by means of a roll molding type dry granulating machine to produce a molded product, and granules were produced from the molded product by means of an oscillator type grinder. 19.15 kg of the shaped granules and 0.20 kg of calcium stearate were mixed together by means of a container type mixer to obtain granules to be tableted (see Table 4). The physical properties of the granules to be tableted are shown in Table 5.

Comparative Example 2: (330 mg preparation)

17.17 kg of magnesium oxide particles having an average secondary particle diameter of 6.50 μm, 1.20 kg of crystalline cellulose-I, 0.36 kg of corn starch and 0.59 kg of croscarmellose sodium were mixed together by means of a container type mixer, the resulting mixture was granulated at a roll pressure of 5 MPa by means of a roll molding type dry granulating machine to produce a molded product, and granules were produced from the molded product by means of an oscillator type grinder. 19.3 kg of the shaped granules and 0.21 kg of calcium stearate were mixed together by means of a container type mixer to obtain granules to be tableted (see Table 4). The physical properties of the granules to be tableted are shown in Table 6.

Comparative Example 3: (250 mg preparation)

17.17 kg of magnesium oxide particles having an average secondary particle diameter of 6.50 μm, 1.24 kg of crystalline cellulose-I, 0.34 kg of corn starch and 0.62 kg of croscarmellose sodium were mixed together by means of a container type mixer, the resulting mixture was granulated at a roll pressure of 5 MPa by means of a roll molding type dry granulating machine to obtain a molded product, and granules were produced from the molded product by means of an oscillator type grinder. 19.37 kg of the shaped granules and 0.21 kg of calcium stearate were mixed together by means of a container type mixer to obtain granules to be tableted (see Table 4). The physical properties of the granules to be tableted are shown in Table 7.

Table 4

Features of production processes

| Production process (Comparative Examples) | Production process (Examples) |
|---|---|
| Raw materials except for calcium stearate are mixed together, granulated and mixed with a lubricant to produce granules to be tableted. | Raw materials to be contained in granules are mixed together, granulated, mixed with a lubricant and externally mixed with a functional additive to produce granules to be tableted. |

Table 5

Physical properties of granules to be tableted to produce 500 mg preparations

| | Pharmaceutical formulation | | |
|---|---|---|---|
| | Ex. 1 | Ex. 2 | Comp. Ex. 1 |
| Physical properties of granules | | | |
| Bulk density (g/ml) | 0.622 | 0.631 | 0.617 |
| Particle size | | | |
| 710 μm on (%) | 11.57 | 22.09 | 10.78 |
| 106 μm pass (%) | 13.02 | 12.01 | 8.54 |
| Average particle diameter D50 (μm) | 410.39 | 517.38 | 427.92 |

Table 6

Physical properties of granules to be tableted to produce 330 mg preparations

| | Pharmaceutical formulation | | |
|---|---|---|---|
| | Ex. 3 | Ex. 4 | Comp. Ex. 2 |
| Physical properties of granules | | | |

(continued)

Physical properties of granules to be tableted to produce 330 mg preparations

| | Pharmaceutical formulation | | |
|---|---|---|---|
| | Ex. 3 | Ex. 4 | Comp. Ex. 2 |
| Bulk density (g/ml) | 0.664 | 0.631 | 0.619 |
| Particle size | | | |
| 710 μm on (%) | 14.80 | 22.09 | 11.36 |
| 106 μm pass (%) | 10.87 | 12.01 | 9.85 |
| Average particle diameter D50 (μm) | 467.49 | 517.38 | 453.76 |

Table 7

Physical properties of granules to be tableted to produce 250 mg preparations

| | Pharmaceutical formulation | | |
|---|---|---|---|
| | Ex. 5 | Ex. 6 | Comp. Ex. 3 |
| Physical properties of granules | | | |
| Bulk density (g/ml) | 0.669 | 0.631 | 0.645 |
| Particle size | | | |
| 710 μm on (%) | 14.39 | 22.09 | 13.89 |
| 106 μm pass (%) | 14.02 | 12.01 | 9.68 |
| Average particle diameter D50 (μm) | 441.22 | 517.38 | 440.85 |

Examples 7 and 8 and Comparative Example 4 (500 mg preparations)

The granules produced in Examples 1 and 2 were tableted by a rotary tableting machine having 24 corner single-R pestles with a diameter of 10.5 mm effective for suppressing edge wear (corner angle of 30°, corner horizontal length of 0.8 mm, cup depth of 0.94 mm, R of 20°) at a tableting pressure of 15 to 16 KN to obtain magnesium oxide tablets having a weight of 570 mg and a thickness of 5.1 mm. The granules produced in Comparative Example 3 were tableted by using a single-R pestle (diameter of 10.5 mm, cup depth of 0.78 mm, R of 17.5) to obtain a magnesium oxide tablet having a weight of 580 mg and a thickness of 5.1 mm (Comparative Example 3). The term "single-R" means a shape having no corner angle and no corner horizontal length. The physical properties of the tablet are shown in Table 8.

Table 8

Physical properties of tablets of 500 mg preparations

| | | Ex. 7 | Ex. 8 | Comp. Ex. 4 |
|---|---|---|---|---|
| Tablet Example No. | | Ex. 1 | Ex. 2 | Comp. Ex. 1 |
| | Shape of pestle | Corner single-R | Corner single-R | Single-R |
| Physical properties of tablet | | | | |
| | Hardness of tablet (N) | 108 | 121 | 101 |
| | Disintegration time (seconds) | 8 | 7 | 9 |
| | Degree of friability (%) | 0.29 | 0.20 | 1.05 |

(continued)

Physical properties of tablets of 500 mg preparations

|  |  | Ex. 7 | Ex. 8 | Comp. Ex. 4 |
|---|---|---|---|---|
| Tablet Example No. |  | Ex. 1 | Ex. 2 | Comp. Ex. 1 |
|  | Shape of pestle | Corner single-R | Corner single-R | Single-R |
|  | Existence of tableting troubles such as capping, sticking, etc. | none | none | none |

Examples 9 and 10 and Comparative Example 5 (330 mg preparations)

The granules produced in Examples 3 and 4 were tableted by a rotary tableting machine having 24 corner single-R pestles with a diameter of 9.0 mm effective for suppressing edge wear (corner angle of 30°, corner horizontal length of 0. 68 mm, cup depth of 0.87 mm, R of 14.7) at a tableting pressure of 9 to 10 KN to obtain magnesium oxide tablets having a weight of 376 mg and a thickness of 4.7 mm.
The granules produced in Comparative Example 2 were tableted by using a single-R pestle (diameter of 9.0 mm, cup depth of 0.77 mm, R of 13 mm) to obtain a magnesium oxide tablet having a weight of 375 mg and a thickness of 4.7 mm (Comparative Example 5). The physical properties of these tablets are shown in Table 9.

Table 9

Physical properties of tablets of 330 mg preparations

|  |  | | Ex. 9 | Ex. 10 | Comp. Ex. 5 |
|---|---|---|---|---|---|
| Tablet Example No. |  | | Ex. 3 | Ex. 4 | Comp. Ex. 2 |
|  | Shape of pestle | | Corner single-R | Corner single-R | Single-R |
|  | Physical properties of tablet | | | | |
|  |  | Hardness of tablet (N) | 84 | 87 | 79 |
|  |  | Disintegration time (seconds) | 8 | 7 | 8 |
|  |  | Degree of friability (%) | 0.07 | 0.03 | 0.59 |
|  | Existence of tableting troubles such as capping, sticking, etc. | | none | none | none |

Examples 11 and 12 and Comparative Example 6 (250 mg preparations)

The granules produced in Examples 5 and 6 were tableted by a rotary tableting machine having 24 corner single-R pestles with a diameter of 8.0 mm effective for suppressing edge wear (corner angle of 30°, corner horizontal length of 0. 61 mm, cup depth of 0.78 mm, R of 12.8) at a tableting pressure of 9 to 10 KN to obtain magnesium oxide tablets having a weight of 285 mg and a thickness of 4.4 mm.
The granules produced in Comparative Example 3 were tableted by using a single-R pestle (diameter of 8.0 mm, cup depth of 0.65 mm, R of 12) to obtain a magnesium oxide tablet having a weight of 285 mg and a thickness of 4.4 mm (Comparative Example 6). The physical properties of these tablets are shown in Table 10.

Table 10

Physical properties of tablets of 250 mg preparations

|  |  | Ex. 11 | Ex. 12 | Comp. Ex. 6 |
|---|---|---|---|---|
| Tablet Example No. |  | Ex. 5 | Ex. 6 | Comp. Ex. 3 |
|  | Shape of pestle | Corner single-R | Corner single-R | Single-R |
|  | Physical properties of tablet | | | |

(continued)

Physical properties of tablets of 250 mg preparations

| Tablet Example No. | | | Ex. 11 | Ex. 12 | Comp. Ex. 6 |
|---|---|---|---|---|---|
| | | | Ex. 5 | Ex. 6 | Comp. Ex. 3 |
| Shape of pestle | | | Corner single-R | Corner single-R | Single-R |
| | | Hardness of tablet (N) | 75 | 87 | 76 |
| | | Disintegration time (seconds) | 8 | 6 | 7 |
| | | Degree of friability (%) | 0.07 | 0.01 | 0.44 |
| Existence of tableting troubles such as capping, sticking, etc. | | | none | none | none |

Examples 13 to 15 and Comparative Examples 7 to 9 (influence upon physical properties of tablet of tablet shape)

Tables 11 to 13 show the relationship between a combination of pharmaceutical formulation and the tablet-like shape of a pestle (single-R and corner single-R) and the physical properties (hardness and degree of friability) of each tablet. As a result, 250 mg, 330 mg and 500 mg preparations experienced a reduction in the degree of friability when they had the tablet shape of the present invention, which proves their superiority. The degree of friability was greatly reduced by changing the pestle to a corner single-R pestle.

Table 11

Tablet hardness (N) and degree of friability (%) of 250 mg preparation

| | Pharmaceutical formulation | | | |
|---|---|---|---|---|
| | Ex. 13 | | Comp. Ex. 7 | |
| | hardness (N) | degree of friability (%) | hardness (N) | degree of friability (%) |
| Shape of pestle | | | | |
| Single-R pestle | 79 | 0.17 | 68 | 0.44 |
| Corner single-R pestle | 76 | 0.03 | 74 | 0.17 |

Table 12

Tablet hardness (N) and degree of friability (%) of 330 mg preparation

| | Pharmaceutical formulation | | | |
|---|---|---|---|---|
| | Ex. 14 | | Comp. Ex. 8 | |
| | hardness (N) | degree of friability (%) | hardness (N) | degree of friability (%) |
| Shape of pestle | | | | |
| Single-R pestle | 90 | 0.27 | 79 | 0.59 |
| Corner single-R pestle | 103 | 0.08 | 86 | 0.25 |

Table 13

Tablet hardness (N) and degree of friability (%) of 500 mg preparation

| | Pharmaceutical formulation | | | |
| | Ex. 15 | | Comp. Ex. 9 | |
| | hardness (N) | degree of friability (%) | hardness (N) | degree of friability (%) |
|---|---|---|---|---|
| Shape of pestle | | | | |
| Single-R pestle | 119 | 0.68 | 105 | 1.12 |
| Corner single-R pestle | 134 | 0.13 | 101 | 0.72 |

Examples 16 and 17 and Comparative Example 10 (studies on the suppression of extension of disintegration time at the time of acceleration of bare tablet)

Tablets of Examples 7 and 8 and Comparative Example 4 were used to carry out the evaluation of stability. The suppression of the extension of the disintegration time of a bare tablet was measured under acceleration conditions (40°C, RH 75%). The results are shown in Table 14.

Table 14

Change of disintegration time under acceleration conditions (40°C, RH75%) of bare tablet (500 mg preparation)

| | Ex. 16 | Ex. 17 | Comp. Ex. 10 |
| Preparation Example No. | Ex. 7 | Ex. 8 | Comp. Ex. 4 |
| | disintegration time (seconds) | disintegration time (seconds) | disintegration time (seconds) |
|---|---|---|---|
| Number of days of acceleration | | | |
| Initial | 9 | 7 | 9 |
| 1 day | 7 | - | 8 |
| 2 days | - | 9 | 15 |
| 3 days | - | 14 | 52 |
| 4 days | 64 | 21 | 156 |
| 5 days | 87 | - | - |
| 6 days | 109 | - | - |
| 1 week | 121 | 55 | >600 |
| 2 weeks | 297 | 86 | - |
| 3 weeks | - | 110 | - |
| 1 month | 467 | 128 | >1800 |

Examples 18 and 19 and Comparative Example 11 (evaluation of cracking and chipping of tablet in drop test)

A bottle containing tablets was dropped vertically from the top of a desk at a height of 80 cm to check whether the tablets were cracked or chipped. The results are shown in Table 15. As a result, the cracking and chipping of the tablets of the present invention were greatly suppressed.

**EP 2 476 423 A1**

Table 15
Bottle drop test (500 mg preparations)

| | Ex. 18 | Ex. 19 | Comp. Ex. 11 |
|---|---|---|---|
| Preparation Example No. | Ex. 7 | Ex. 8 | Comp. Ex. 4 |
| Cracked (tablet) | 18 | 6 | 37 |
| Chipped (tablet) | 13 | 7 | 17 |
| Total number (of tablets) | 31 | 13 | 54 |

**Claims**

1. An antacid and laxative tablet comprising magnesium oxide particles as the main component, wherein

   (a) the average secondary particle diameter measured by a laser diffraction scattering method of the magnesium oxide particles is 0.5 to 10 $\mu$m, (b) the content of the magnesium oxide particles is 85 to 96 wt%, (c) the content of crystalline cellulose as a binder is 2 to 8 wt%, (d) the content of croscarmellose sodium as a disintegrating agent-I is 0.8 to 2. 5 wt%, (e) the content of insoluble polyvinyl pyrrolidone as a disintegrating agent-II is 1 to 3.5 wt%, and (f) the content of a lubricant is 0.5 to 2 wt%.

2. The antacid and laxative tablet according to claim 1, wherein the magnesium oxide particles have an average secondary particle diameter measured by the laser diffraction scattering method of 1 to 7 $\mu$m.

3. The antacid and laxative tablet according to claim 1, wherein the content of the magnesium oxide particles is 88 to 92 wt%.

4. The antacid and laxative tablet according to claim 1, wherein the content of crystalline cellulose as the binder is 4 to 6 wt%.

5. The antacid and laxative tablet according to claim 1, wherein the content of croscarmellose sodium as the disintegrating agent-I is 1.2 to 1.8 wt%.

6. The antacid and laxative tablet according to claim 1, wherein the content of insoluble polyvinyl pyrrolidone as the disintegrating agent-II is 1.5 to 2.8 wt%.

7. The antacid and laxative tablet according to claim 1, wherein the lubricant is calcium stearate.

8. The antacid and laxative tablet according to claim 1 which has a domed shape on each of the upper and lower horizontal surfaces of a cylindrical shape, the domed shape on each of the upper and lower horizontal surfaces satisfying the following requirements (a), (b) and (c) in the cross-section shape including the center line of the cylindrical shape:

   (a) the corner angle should be 25 to 45°,
   (b) the corner horizontal length should be 0.30 to 1.0 mm, and
   (c) the cup depth should be 0.6 to 1.2 mm.

9. The antacid and laxative tablet according to claim 8, wherein the corner angle (a) is 28 to 40°.

10. The antacid and laxative tablet according to claim 8, wherein the corner horizontal length (b) is 0.35 to 0.85 mm.

11. The antacid and laxative tablet according to claim 8, wherein the cup depth (c) is 0.65 to 1.1 mm.

12. The antacid and laxative tablet according to claim 8, wherein the length (diameter) of the horizontal surface in the cross-section shape is 7 to 11 mm.

13. A process of producing an antacid and laxative tablet comprising magnesium oxide particles as the main component, comprising the steps of:

   (1) mixing together magnesium oxide particles having an average secondary particle diameter measured by a laser diffraction scattering method of 0.5 to 10 $\mu$m, crystalline cellulose as a binder, croscarmellose sodium as a disintegrating agent-I and a lubricant, and dry granulating the resulting mixture to produce magnesium oxide granules;
   (2) blending crystalline cellulose as a binder, insoluble polyvinyl pyrrolidone as a disintegrating agent-II and a lubricant with the magnesium oxide granules; and
   (3) tableting the obtained granules.

14. The process of producing an antacid and laxative tablet according to claim 13, wherein the magnesium oxide granules have an average particle diameter of 250 to 550 $\mu$m.

15. Use of the tablet of claim 1 or 13 for antacid or laxative purpose.

Fig. 1

**INTERNATIONAL SEARCH REPORT**

| International application No. |
|---|
| PCT/JP2010/064066 |

A. CLASSIFICATION OF SUBJECT MATTER
*A61K33/08*(2006.01)i, *A61K9/20*(2006.01)i, *A61K47/12*(2006.01)i, *A61K47/32*(2006.01)i, *A61K47/38*(2006.01)i, *A61P1/04*(2006.01)i, *A61P1/10*(2006.01)i

According to International Patent Classification (IPC) or to both national classification and IPC

B. FIELDS SEARCHED

Minimum documentation searched (classification system followed by classification symbols)
A61K9/00-9/72, A61K33/08, A61K47/00-47/48, A61P1/00-1/18

Documentation searched other than minimum documentation to the extent that such documents are included in the fields searched
Jitsuyo Shinan Koho          1922-1996     Jitsuyo Shinan Toroku Koho     1996-2010
Kokai Jitsuyo Shinan Koho    1971-2010     Toroku Jitsuyo Shinan Koho     1994-2010

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2003-146889 A (Kyowa Chemical Industry Co., Ltd.), 21 May 2003 (21.05.2003), entire text & US 7147868 B2 & EP 1421944 A1 & WO 2003/018034 A1 | 1-14 |
| Y | WO 2007/074910 A1 (Takeda Chemical Industries, Ltd.), 05 July 2007 (05.07.2007), paragraphs [0054], [0072] to [0073] & US 2009/0104264 A1 & EP 1967184 A1 | 1-14 |

☒ Further documents are listed in the continuation of Box C.      ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 September, 2010 (01.09.10) | 14 September, 2010 (14.09.10) |

| Name and mailing address of the ISA/ Japanese Patent Office | Authorized officer |
|---|---|
| Facsimile No. | Telephone No. |

Form PCT/ISA/210 (second sheet) (July 2009)

**EP 2 476 423 A1**

**INTERNATIONAL SEARCH REPORT**

| International application No. |
| --- |
| PCT/JP2010/064066 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2006-505566 A  (Orexo AB.),<br>16 February 2006 (16.02.2006),<br>paragraphs [0095] to [0097]<br>& US 2006/0115530 A1    & US 2007/0031497 A1<br>& US 2010/0151013 A    & EP 1558288 A1<br>& WO 2004/035090 A1 | 1-14 |
| Y | JP 8-208521 A  (Rhone-Poulenc Rorer GmbH),<br>13 August 1996 (13.08.1996),<br>paragraphs [0018], [0036], [0039]<br>& EP 715846 A1 | 1-15 |
| Y | WO 2007/007659 A1  (Takeda Chemical Industries,<br>Ltd.),<br>18 January 2007 (18.01.2007),<br>claims; paragraphs [0007] to [0013], [0056],<br>[0067]; fig. 3<br>& US 2009/0148520 A1    & EP 1905430 A1<br>& CA 2614547 A | 9-12 |
| Y | JP 49-037026 B1  (Tanabe Seiyaku Co., Ltd.),<br>04 October 1974 (04.10.1974),<br>table 1; page 3; fig. 2<br>(Family: none) | 9-12 |
| Y | Masao UENO, "Kineusu no Sekkei to Seisaku",<br>Pharm Tech Japan, 1995, vol.11, no.5, pages 549<br>to 552 | 9-12 |
| Y | Kazuo KIGASAWA et al., "Pharmaceutical Studies<br>on Physical Properties of Solid Form Drugs.<br>III. Influence of Tablet Formula and Tablet<br>Shape on Distribution of Boring Hardness inside<br>Tablet", Journal of the Pharmaceutical Society<br>of Japan, 1975, vol.95, no.7, pages 769 to 773 | 9-12 |

Form PCT/ISA/210 (continuation of second sheet) (July 2009)

19

## INTERNATIONAL SEARCH REPORT

| International application No. |
| --- |
| PCT/JP2010/064066 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 15
   because they relate to subject matter not required to be searched by this Authority, namely:
   Claim 15 pertains to a method for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003146889 A